# EUROPEAN PATENT APPLICATION

(11) **EP 3 345 550 A1**
(43) Date of publication of application: **11.07.2018**
(21) Application number: 16841182.5
(22) Date of filing: 12.04.2016
(51) Int. Cl.: A61B 8/12, A61B 1/00, A61B 1/06

(54) **ENDOSCOPE CONNECTOR**

(30) Priority: 31.08.2015 JP 2015170676
(71) Applicant: Olympus Corporation, Hachioji-shi Tokyo 192-8507 (JP)
(72) Inventor: SAIGA, Kazuya, Hachioji-shi Tokyo 192-8507 (JP)
(74) Representative: Gunzelmann, Rainer
(86) International application number: PCT/JP2016/061806
(87) International publication number: WO 2017/038150

(57) **Abstract**

An endoscope connector includes an outer casing, an electrical connector attached to an attachment hole of the outer casing, a first metal member disposed in the outer casing, a second metal member attached to the electrical connector, an insulating member main body 661 placed between the first and second metal members, and electrically insulates the first and second metal members from each other, a capacitor 67 fixed to the insulating member main body 661, a first conduction member 68 fixed to the insulating member main body 661 and electrically connected to one leg 671 of the capacitor 67, the first conduction member contacting the first metal member and being electrically connected to the first metal member, and a second conduction member fixed to the insulating member main body 661 and electrically connected to another leg of the capacitor 67, the second conduction member contacting the second metal member and being electrically connected to the second metal member.

## Description

### Field

The present invention relates to an endoscope connector.

### Background

There has been conventionally known an endoscope system in which an ultrasound endoscope including an insertion portion to be inserted into a subject, an ultrasound observation apparatus that processes a signal from an ultrasound transducer provided at a distal end of the insertion portion, and an endoscope observation apparatus that processes a signal from an image sensor provided at the distal end of the insertion portion are connected via an endoscope connector (for example, refer to Patent Literature 1).

The endoscope connector (connector portion) described in Patent Literature 1 includes an outer casing having an attachment hole, and an ultrasound connector attached to the attachment hole. In addition, a first metal member (connector portion main body) being a metal connected to a patient GND on the endoscope observation apparatus side, and a second metal member (metal member) being a grounding GND metal on the ultrasound observation apparatus side that is attached to the ultrasound connector are provided in the outer casing. In addition, the first and second metal members are electrically-isolated from each other, and electrically connected via a capacitor in a high-frequency manner, for causing an ultrasound image generated by the ultrasound observation apparatus, to be a good image with reduced noise, and assuring electrical safety.

### Citation List

### Patent Literature

Patent Literature 1: JP 2007-185392 A

### Summary

### Technical Problem

Nevertheless, in the endoscope connector described in Patent Literature 1, when the first and second metal members are electrically connected via the capacitor in a high-frequency manner, the capacitor and the first metal member, and the capacitor and the second metal member are connected by respective lead wires. Thus, in assembling the endoscope connector, it is necessary to perform a connecting work for the lead wires. This causes such a problem that it is difficult to enhance assembling workability.

The present invention has been contrived in view of the foregoing, and an object of the present invention is to provide an endoscope connector that can enhance assembling workability.

### Solution to Problem

To solve the above-described problem and achieve the object, an endoscope connector according to the present invention includes: an outer casing including an attachment hole configured to communicate between an inside and an outside of the outer casing; an electrical connector attached to the attachment hole; a first metal member disposed in the outer casing; a second metal member attached to the electrical connector; an insulating member placed between the first metal member and the second metal member in a state in which the electrical connector is attached to the attachment hole, the insulating member electrically insulating the first metal member and the second metal member from each other; a capacitor fixed to the insulating member; a first conduction member fixed to the insulating member and electrically connected to one leg of the capacitor, the first conduction member contacting the first metal member to be electrically connected to the first metal member in the state in which the electrical connector is attached to the attachment hole; and a second conduction member fixed to the insulating member and electrically connected to another leg of the capacitor, the second conduction member contacting the second metal member to be electrically connected to the second metal member in the state in which the electrical connector is attached to the attachment hole.

Moreover, in the above-described endoscope connector according to the present invention, the first conduction member is formed by a finger.

Moreover, in the above-described endoscope connector according to the present invention, the second conduction member is formed by a finger.

Moreover, in the above-described endoscope connector according to the present invention, the insulating member includes a coating portion configured to coat at least the one leg or the other leg of the capacitor.

Moreover, in the above-described endoscope connector according to the present invention, a thickness of the coating portion is 0.4 mm or more.

Moreover, in the above-described endoscope connector according to the present invention, the insulating member is formed in a container shape in which part of the electrical connector is accommodated, and the second conduction member contacts the second metal member fixed to the electrical connector and is electrically connected to the second metal member in a state in which the part of the electrical connector is accommodated in the insulating member.

### Advantageous Effects of Invention

In the endoscope connector according to the present invention, the first and second metal members are electrically insulated from each other by interposing the insulating member between the first and second metal members. In addition, by fixing the capacitor and the first and second conduction members to the insulating member, when the electrical connector is attached to the attachment hole, the first and second metal members are electrically connected via the capacitor in a high-frequency manner.

Thus, the endoscope connector according to the present invention brings about such an effect that the first and second metal members can be electrically connected via the capacitor in a high-frequency manner only by attaching the electrical connector to the attachment hole, and assembling workability can be enhanced.

### Brief Description of Drawings

FIG. 1 is a diagram schematically illustrating an endoscope system according to an embodiment of the present invention.
FIG. 2 is a diagram illustrating a wiring state of an imaging cable in an operating unit illustrated in FIG. 1.
FIG. 3 is perspective view of an endoscope connector illustrated in FIG. 1, viewed from a front side and an upper left side.
FIG. 4 is a perspective view illustrating a state in which an ultrasound connector is removed from the endoscope connector illustrated in FIG. 3.
FIG. 5 is a diagram illustrating a wiring state of a US cable in an outer casing illustrated in FIGS. 3 and 4.
FIG. 6 is a diagram illustrating a wiring state of a US cable in the outer casing illustrated in FIGS. 3 and 4.
FIG. 7 is a perspective view of the ultrasound connector removed from the endoscope connector illustrated in FIG. 3, viewed from an outer casing inner side.
FIG. 8 is a perspective view illustrating a state in which a second metal member is attached to the ultrasound connector illustrated in FIG. 7.
FIG. 9 is a perspective view illustrating a state in which an insulating member is attached to the ultrasound connector illustrated in FIG. 8.
FIG. 10 is a perspective view of an insulating member main body illustrated in FIG. 9, viewed from an outer casing inner side.
FIG. 11 is a perspective view of the insulating member main body illustrated in FIG. 9, viewed from an ultrasound connector side.

### Description of Embodiments

A mode for carrying out the present invention (hereinafter, referred to as an embodiment) will be described below with reference to the drawings. In addition, the present invention is not limited by the embodiment to be described below. Furthermore, in the description of the drawings, the same parts are assigned the same signs.

### [Schematic Configuration of Endoscope System]

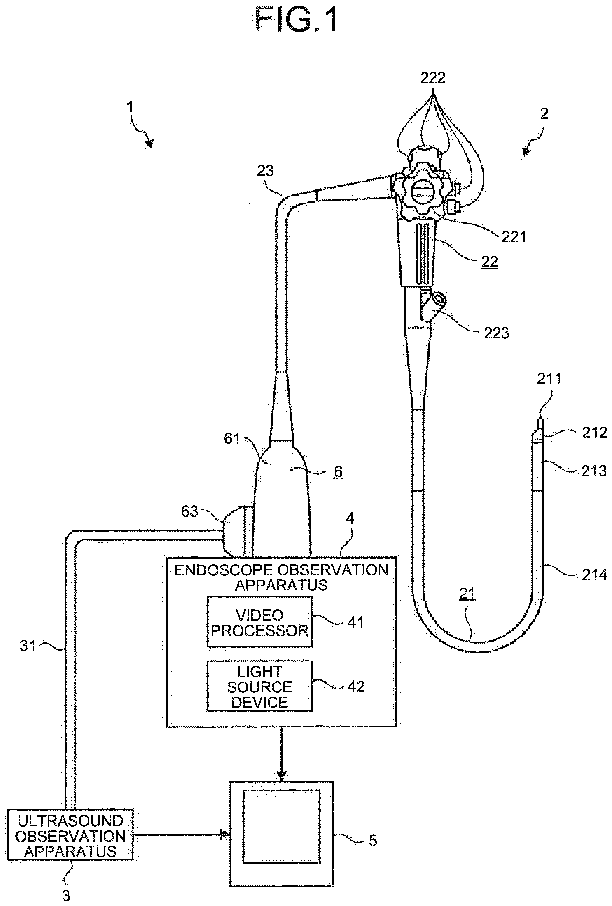

FIG. 1 is a diagram schematically illustrating an endoscope system 1 according to an embodiment of the present invention.

The endoscope system 1 is a system for performing ultrasound diagnosis of the inside of a subject such as a human, using an ultrasound endoscope. As illustrated in FIG. 1, the endoscope system 1 includes an endoscope 2, an ultrasound observation apparatus 3, an endoscope observation apparatus 4, and a display device 5.

Part of the endoscope 2 can be inserted into a subject, and the endoscope 2 is an ultrasound endoscope having a function of transmitting an ultrasound pulse toward a body wall on the inside of the subject, receiving an ultrasound echo reflected by the subject, and outputting an echo signal, and a function of imaging the inside of the subject to output an image signal.

In addition, a detailed configuration of the endoscope 2 will be described later.

The ultrasound observation apparatus 3 electrically-connects to the endoscope 2 via an ultrasound cable 31 (FIG. 1), outputs a pulse signal to the endoscope 2 via the ultrasound cable 31, and inputs an echo signal from the endoscope 2. Then, the ultrasound observation apparatus 3 performs predetermined processing on the echo signal to generate an ultrasound image.

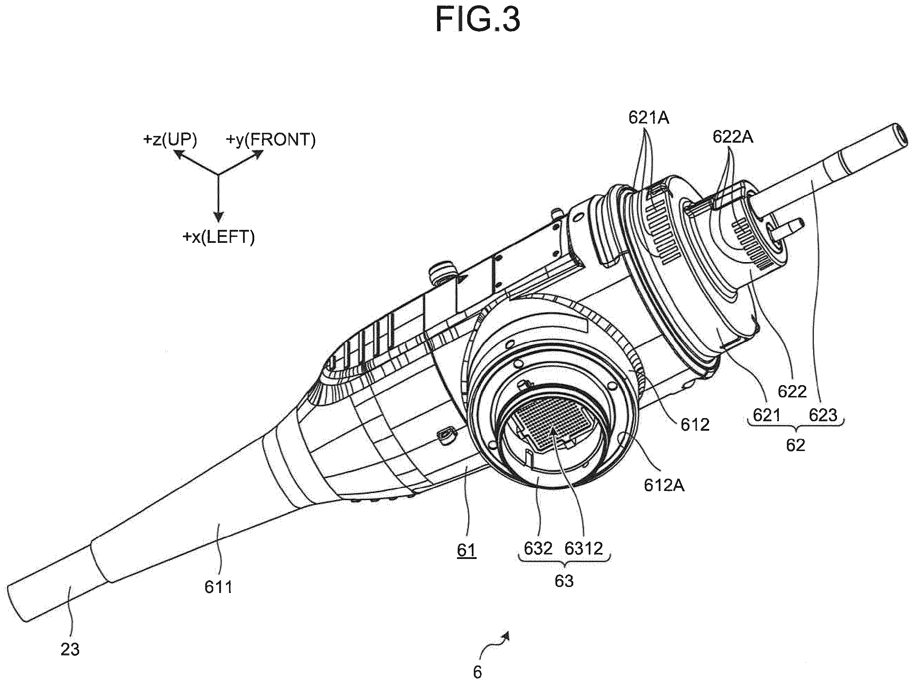

An endoscope connector 6 (refer to FIG. 3) of the endoscope 2 that is to be described later is detachably connected to the endoscope observation apparatus 4. As illustrated in FIG. 1, the endoscope observation apparatus 4 includes a video processor 41 and a light source device 42.

The video processor 41 outputs a control signal to the endoscope 2 via the endoscope connector 6, and inputs an image signal from the endoscope 2 via the endoscope connector 6. Then, the video processor 41 performs predetermined processing on the image signal to generate an endoscope image.

The light source device 42 supplies, via the endoscope connector 6, the endoscope 2 with illumination light for illuminating the inside of the subject.

The display device 5 is formed by using a liquid crystal or an organic electro luminescence (EL), and displays the ultrasound image generated in the ultrasound observation apparatus 3, the endoscope image generated in the endoscope observation apparatus 4, and the like.

### [Configuration of Endoscope]

As illustrated in FIG. 1, the endoscope 2 includes an insertion portion 21, an operating unit 22, a universal cable 23, and the endoscope connector 6.

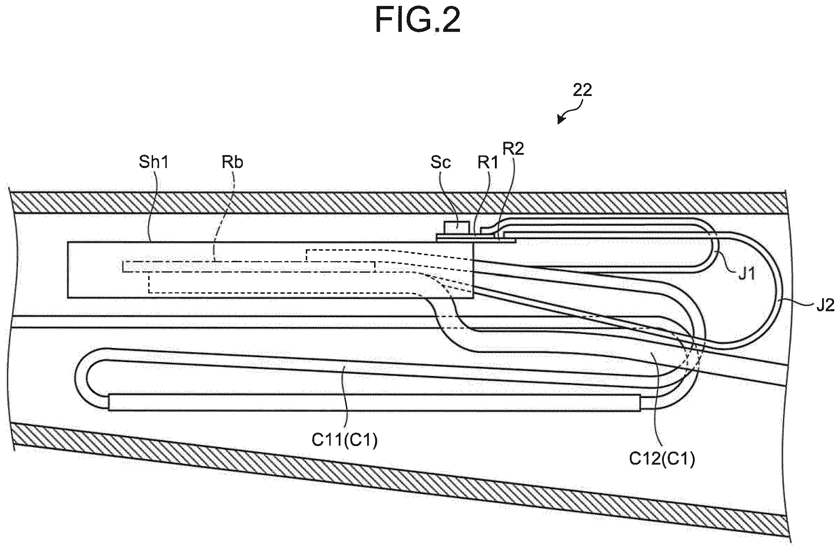

Here, a light guide for transmitting illumination light supplied from the light source device 42, a US cable for ultrasound observation (for the transmission of a pulse signal and an echo signal), an imaging cable C1 (refer to FIG. 2) for endoscope observation (for the transmission of a control signal, an image signal, and the like), and the like are laid in the endoscope 2 (in the insertion portion 21, the operating unit 22, the universal cable 23, and the endoscope connector 6), though these are not specifically illustrated in the drawing.

The insertion portion 21 is a portion to be inserted into the subject. As illustrated in FIG. 1, the insertion portion 21 includes an ultrasound probe 211 provided at a distal end, a rigid member 212 connected to a proximal end side (the operating unit 22 side) of the ultrasound probe 211, a curve portion 213 capable of curving that is connected to a proximal end side of the rigid member 212, and a flexible tube portion 214 having flexibility that is connected to a proximal end side of the curve portion 213.

Here, in addition to the aforementioned light guide, the US cable, and the imaging cable C1 (refer to FIG. 2), processing tool tubes into which various processing tools (e.g., puncture needle, etc.) are inserted, and the like are laid in the insertion portion 21 (the rigid member 212, the curve portion 213, and the flexible tube portion 214), though these are not specifically illustrated in the drawing.

In the example illustrated in FIG. 1, the ultrasound probe 211 is a convex-shaped ultrasound probe, and has a configuration in which a plurality of ultrasound transducers (not illustrated) are regularly-arrayed in such a manner as to form a convex-shaped arc.

Here, the ultrasound transducer includes an acoustic lens, a piezoelectric element, and a matching layer, and acquires an ultrasound echo contributing to an ultrasound tomographic image of the inside of the body wall on the inside of the subject.

Then, the ultrasound probe 211 converts a pulse signal input from the ultrasound observation apparatus 3 via the aforementioned US cable and the ultrasound cable 31, into an ultrasound pulse, and transmits the ultrasound pulse into the subject. In addition, the ultrasound probe 211 converts an ultrasound echo reflected inside the subject, into an electrical echo signal, and outputs the electrical echo signal to the ultrasound observation apparatus 3 via the above-described US cable and the ultrasound cable 31.

The rigid member 212 is a hard member formed of resin material, and has a substantially-circular cylindrical shape.

Here, an observation window, an illumination window, a processing tool passage, and the like are formed in the rigid member 212, though these are not specifically illustrated in the drawing.

The observation window, the illumination window, and the processing tool passage are holes penetrating through from the proximal end (end portion on the operating unit 22 side) of the rigid member 212 toward a distal end, and more specifically, have the following functions.

The observation window is a hole for acquiring an optical image of the inside of the subject. In addition, an image sensor (not illustrated) that is connected to the aforementioned imaging cable C1 (refer to FIG. 2), and outputs an image signal according to the optical image of the inside of the subject, and an objective lens (not illustrated) are inserted into the observation window in a combined state. The image signal output from the image sensor is transmitted to the endoscope observation apparatus 4 (the video processor 41) via the aforementioned imaging cable C1 (refer to FIG. 2).

The illumination window is a hole for emitting illumination light into the subject. In addition, an emission end side of the aforementioned light guide is inserted into the illumination window.

The processing tool passage is a hole for causing the various processing tools to protrude outward. In addition, the aforementioned processing tool tube is connected to the processing tool passage.

The operating unit 22 is a portion that is connected to a proximal end side of the insertion portion 21, and receives various operations from a doctor or the like. As illustrated in FIG. 1, the operating unit 22 includes a curve knob 221 for performing a curving operation of the curve portion 213, and a plurality of operating members 222 for performing various operations.

In addition, a processing tool insertion port 223 that is communicated with the aforementioned processing tool tube, and is provided for inserting the various processing tools into the processing tool tube is formed in the operating unit 22.

FIG. 2 is a diagram illustrating a wiring state of the imaging cable C1 in the operating unit 22.

Here, in the operating unit 22, the imaging cable C1 (FIG. 2) is wired in the following manner.

As illustrated in FIG. 2, the imaging cable C1 includes a first imaging cable C11 and a second imaging cable C12.

One end of the first imaging cable C11 is electrically connected to the aforementioned image sensor, and the other end thereof is electrically connected to a relay board Rb provided in the operating unit 22 while repeating meandering a plurality of times. In addition, the first imaging cable C11 performs transmission of an image signal and a control signal between the aforementioned image sensor and the relay board Rb. In addition, the relay board Rb is illustrated using a dashed-dotted line in FIG. 2, and the relay board Rb has a box shape, and is provided in a shield member Sh1 connected to the same electric potential as a patient GND on the endoscope observation apparatus 4 side.

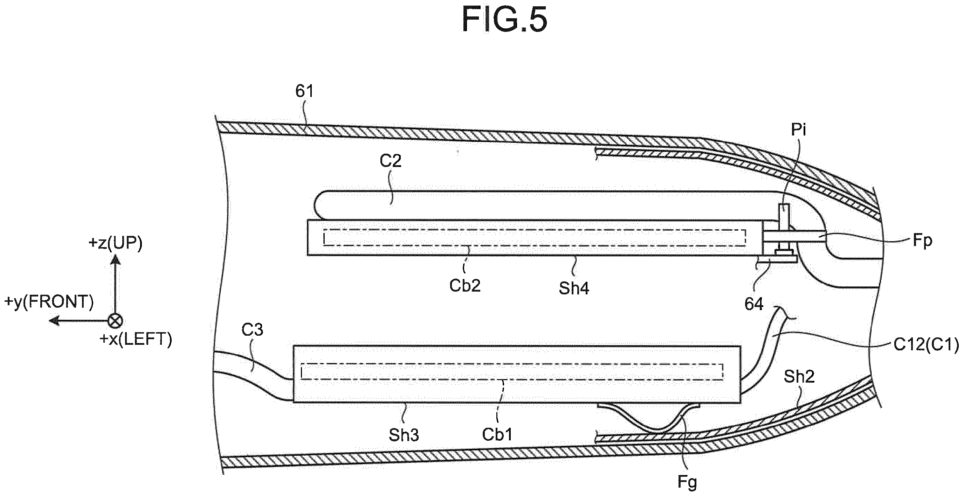

One end of the second imaging cable C12 is electrically connected to the relay board Rb, and the other end thereof is electrically connected to a first circuit board Cb1 (refer to FIG. 5) to be described later that is provided in the endoscope connector 6. In addition, the second imaging cable C12 performs transmission of an image signal and a control signal between the relay board Rb and the first circuit board Cb1.

As illustrated in FIG. 2, respective one ends of jumper lines J1 and J2 are electrically connected to respective shields (e.g., braided shield, etc.) of the aforementioned first and second imaging cables C11 and C12. Then, the respective other ends of the jumper lines J1 and J2 are fastened together by a screw Sc onto the shield member Sh1 via lug terminals R1 and R2.

In other words, the respective shields of the first and second imaging cables C11 and C12 are ensured at the same electric potential, and connected to the same electric potential as the patient GND.

The universal cable 23 is a cable having one end connected to the operating unit 22, and is a cable in which a remote switch cable for transmitting an operation signal according to various operations performed on the operating unit 22 (e.g., operations for instructing freeze, release, and edge enhancement of an endoscope image, an operation for instructing light adjustment of the light source device 42, etc.) is laid in addition to the aforementioned light guide, the US cable, and the imaging cable (the second imaging cable C12).

The endoscope connector 6 is provided at the other end of the universal cable 23, and is a connector for connecting to the ultrasound cable 31 connected to the ultrasound observation apparatus 3, and the endoscope observation apparatus 4 (the video processor 41 and the light source device 42).

### [Configuration of Endoscope Connector]

Next, a configuration of the endoscope connector 6 will be described.

In the following description, based on a posture of when the endoscope connector 6 is connected to the endoscope observation apparatus 4, an upper side in the posture is regarded as "up", a downside in the posture is regarded as "down", a side approaching the endoscope observation apparatus 4 is regarded as "front", a side separated from the endoscope observation apparatus 4 is regarded as "rear", a left side viewed from a front side in the posture is regarded as "left", and a right side viewed from the front side in the posture is regarded as "right".

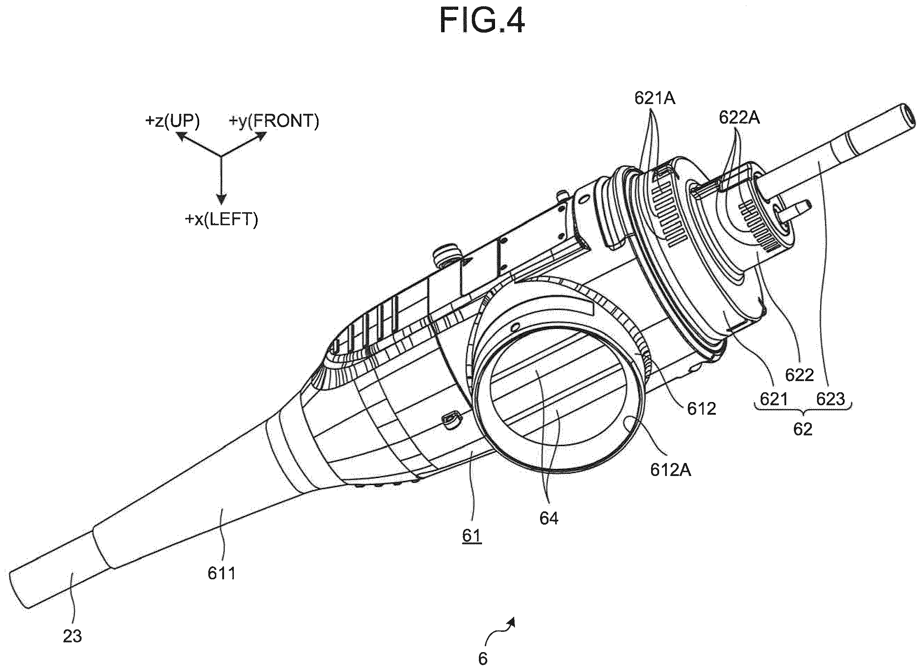

FIG. 3 is perspective view of the endoscope connector 6 viewed from the front side and an upper left side. FIG. 4 is a perspective view illustrating a state in which an ultrasound connector 63 is removed from the endoscope connector 6 illustrated in FIG. 3.

In addition, in FIGS. 3 and 4, for identifying the aforementioned "up and down", "front and rear", and "left and right" of the endoscope connector 6, an XYZ orthogonal coordinate is illustrated. Here, a +Z-axis direction is the "upper direction" of the endoscope connector 6. A +X-axis direction is the "left direction" of the endoscope connector 6. A +Y-axis direction is the "front direction" of the endoscope connector 6.

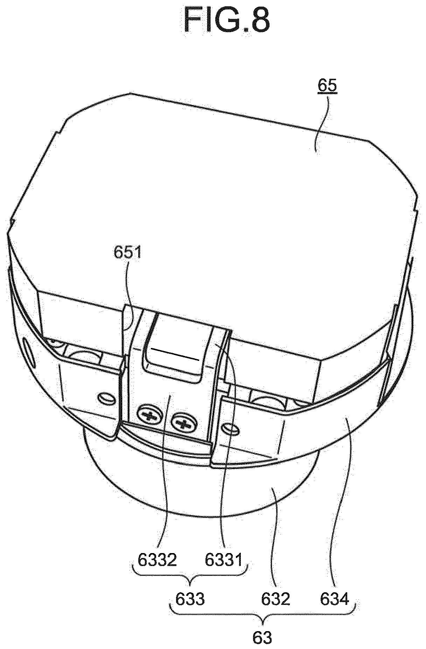

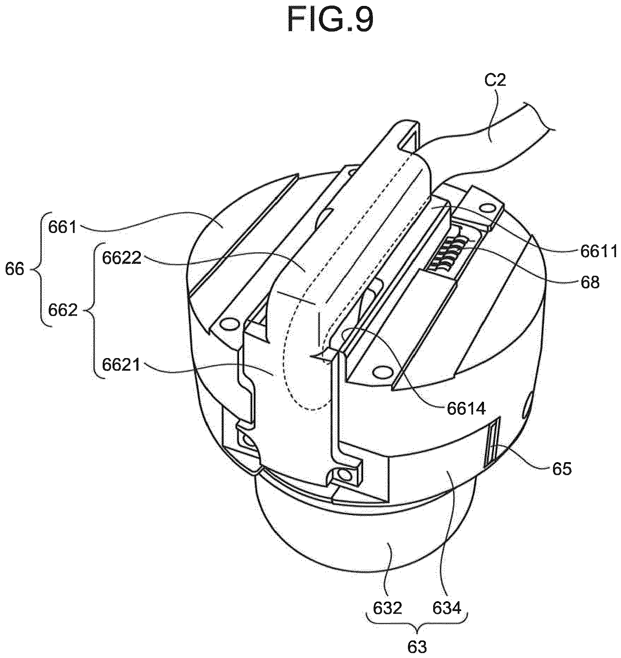

As illustrated in FIG. 3 or 4, the endoscope connector 6 includes an outer casing 61, a plug portion 62, the ultrasound connector 63 (FIG. 3), a first metal member 64 (FIG. 4), a second metal member 65 (refer to FIG. 8), and an insulating member 66 (refer to FIGS. 9 to 11).

As illustrated in FIG. 3 or 4, the outer casing 61 has a substantially-cylindrical shape extending in the front and rear direction (Y-axis direction). In addition, via an aperture portion on the rear side, the universal cable 23 (the aforementioned light guide, the US cable, the imaging cable (the second imaging cable C12), the remote switch cable, etc.) is inserted into the outer casing 61. In addition, as illustrated in FIG. 3 or 4, a bend protection member 611 is provided on the rear side of the outer casing 61.

As illustrated in FIG. 3 or 4, a protruding portion 612 protruding in the X-axis direction is formed on the side surface of the above-described outer casing 61.

The protruding portion 612 is communicated with the inside of the outer casing 61, and has a hollow shape. In addition, as illustrated in FIG. 3 or 4, an attachment hole 612A having an aperture surface positioned on a YZ plane, and communicating the inside and the outside of the outer casing 61 is formed in the protruding portion 612. The attachment hole 612A is a hole to which the ultrasound connector 63 is attached.

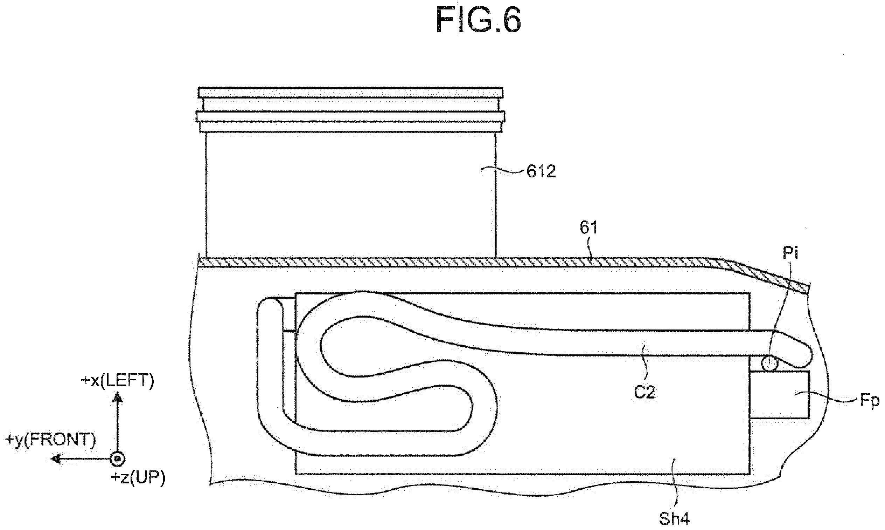

FIGS. 5 and 6 are diagrams illustrating a wiring state of a US cable C2 in the outer casing 61. More specifically, FIG. 5 is a diagram illustrating the wiring state viewed from the right side illustrated in FIG. 3 or 4. FIG. 6 is a diagram illustrating the wiring state viewed from the upper side illustrated in FIG. 3 or 4.

Here, as illustrated in FIG. 5 or 6, the first circuit board Cb1 (FIG. 5), a second circuit board Cb2 (FIG. 5), a shield casing Sh2 (FIG. 5), and the like are provided in the outer casing 61.

The first and second circuit boards Cb1 and Cb2 are supported by the first metal member 64 in the outer casing 61, in a posture substantially-parallel to an XY plane, and in a posture in which they face each other in the Z-axis direction. In addition, in FIGS. 5 and 6, for the convenience of explanation, only part of the first metal member 64 is illustrated, and the other parts are not illustrated in the drawings.

As illustrated in FIG. 5, the first circuit board Cb1 is electrically connected to first and second electrical contact points 621A and 622A of the plug portion 62 via a signal cable C3, and the other end of the second imaging cable C12 is connected thereto. In addition, the first circuit board Cb1 is illustrated using a dashed-dotted line in FIG. 5, and the first circuit board Cb1 has a box shape, and is provided in a shield member Sh3 connected to the same electric potential as the patient GND on the endoscope observation apparatus 4 side.

As illustrated in FIG. 5, the second circuit board Cb2 is provided in the outer casing 61 in a posture parallel to the first circuit board Cb1. In addition, the second circuit board Cb2 is electrically connected to the first circuit board Cb1 so that signal transmission can be performed with the first circuit board Cb1 via a signal cable, and the aforementioned remote switch cable laid from the operating unit 22 to the inside of the outer casing 61 via the universal cable 23 is connected to the second circuit board Cb2, though these are not specifically illustrated in the drawing. More specifically, the remote switch cable is connected to a flexible board Fp (FIG. 5, FIG. 6). In addition, the flexible board Fp is connected to a flexible printed circuits (FPC) connector (not illustrated) mounted on the second circuit board Cb2, from the rear side of the second circuit board Cb2. In addition, the second circuit board Cb2 is illustrated using a dashed-dotted line in FIGS. 5 and 6, and the second circuit board Cb2 has a box shape, and is provided in a shield member Sh4 connected to the same electric potential as the patient GND on the endoscope observation apparatus 4 side.

Then, as illustrated in FIG. 5 or 6, the US cable C2 is provided in the outer casing 61 at a position distant from the first circuit board Cb1, that is, provided on a top surface of the second circuit board Cb2, and is laid toward the ultrasound connector 63 side while repeating meandering a plurality of times.

In other words, the US cable C2 is laid at a position separated from a location where noise is easily generated from the first circuit board Cb1 electrically connected at a position closest to the endoscope observation apparatus 4 when the first and second circuit boards Cb1 and Cb2 are compared, that is, at a position separated from the first circuit board Cb1. Thus, the influence of noise exerted on the US cable C2 can be suppressed, and an ultrasound image can be a good image with a reduced noise.

In the US cable C2, the aforementioned meandering portion, that is, a portion bent at an angle equal to or smaller than at least 90° is coated with a heat shrinkable tube in a not-shrunk state, for preventing damages to the portion. In addition, in the US cable C2, if there is a portion bent at an angle equal to or smaller than at least 90°, such as, for example, a portion laid from the inside of the operating unit 22 to the inside of the universal cable 23, aside from the inside of the outer casing 61, the portion may be similarly coated with a heat shrinkable tube in a not-shrunk state.

Here, as illustrated in FIG. 5 or 6, in the first metal member 64, a fixing pin Pi is attached in a portion positioned on the rear side of the second circuit board Cb2. In addition, the fixing pin Pi is positioned between the flexible board Fp and the US cable C2.

In other words, the fixing pin Pi can prevent mechanical interference between the flexible board Fp and the US cable C2, and prevent the flexible board Fp from being removed from the FPC connector mounted on the second circuit board Cb2.

The shield casing Sh2 is a shield member having a substantially-cylindrical shape following the inner surface of the outer casing 61. In addition, as illustrated in FIG. 5, the shield casing Sh2 is electrically connected to the shield member Sh3 via a finger Fg.

In other words, the shield casing Sh2 is connected to the same electric potential as the patient GND on the endoscope observation apparatus 4 side, and reduces radiation noise generated from the first and second circuit boards Cb1 and Cb2.

The plug portion 62 is a portion to be inserted into the endoscope observation apparatus 4, and connected to the video processor 41 and the light source device 42, and is attached to an aperture portion on the front side of the outer casing 61 as illustrated in FIG. 3 or 4. As illustrated in FIG. 3 or 4, the plug portion 62 includes first and second electrical connector portions 621 and 622, and a light guide cap 623.

As illustrated in FIG. 3 or 4, the first electrical connector portion 621 is positioned on the rearmost side of the plug portion 62, and has a circular cylindrical shape extending in the front and rear direction.

In the first electrical connector portion 621, on part of the outer circumferential surface, a plurality of first electrical contact points 621A is provided in a circumferential direction.

As illustrated in FIG. 3 or 4, the second electrical connector portion 622 is integrally formed on the front side of the first electrical connector portion 621, and has a circular cylindrical shape having an outer dimension smaller than an outer dimension of the first electrical connector portion 621.

In the second electrical connector portion 622, on part of the outer circumferential surface, a plurality of second electrical connector portions 622A is provided in the circumferential direction.

As mentioned above, the above-described plurality of first and second electrical contact points 621A and 622A are electrically connected to the imaging cable C1 and the aforementioned remote switch cable via the signal cable C3 and the first and second circuit boards Cb1 and Cb2. In addition, the plurality of first and second electrical contact points 621A and 622A are electrically connected to the video processor 41 in a state in which the plug portion 62 is inserted into the endoscope observation apparatus 4. In other words, the plurality of first and second electrical contact points 621A and 622A are portions electrically-connecting the imaging cable C1 and the aforementioned remote switch cable, and the video processor 41.

The light guide cap 623 is attached to an end surface on the front side of the second electrical connector portion 622, and protrudes from the end surface on the front side in the +Y-axis direction.

In addition, an incidence end side of the aforementioned light guide is inserted into the light guide cap 623. In addition, the light guide cap 623 connects to the light source device 42 in a state in which the plug portion 62 is inserted into the endoscope observation apparatus 4. In other words, the light guide cap 623 is a portion optically-connecting the aforementioned light guide and the light source device 42.

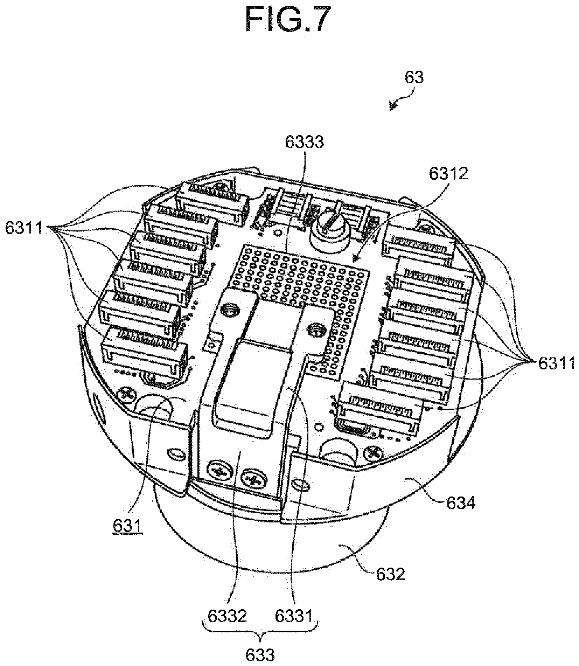

FIG. 7 is a perspective view of the ultrasound connector 63 removed from the endoscope connector 6, viewed from an outer casing 61 inner side.

The ultrasound connector 63 is an electrical connector for electrically-connecting the US cable C2 and the ultrasound cable 31. As illustrated in FIG. 3 or 7, the ultrasound connector 63 includes an ultrasound board 631 (FIG. 7), a frame member 632, an electrical connection member 633 (FIG. 7), and a spacer 634 (FIG. 7).

The ultrasound board 631 has a substantially-circular disc shape, and is a board having a surface (surface on the outer casing 61 inner side) on which a plurality of (12 in the present embodiment) FPC connectors 6311 (FIG. 7) and a plurality of pin terminals 6312 (FIG. 3, FIG. 7) are mounted.

As illustrated in FIG. 6, in the outer casing 61, one end of the US cable C2 laid toward the ultrasound connector 63 side is connected to a plurality of flexible boards (not illustrated). In addition, the plurality of flexible boards (the US cable C2) is connected to the plurality of FPC connectors 6311.

The plurality of pin terminals 6312 are arrayed in a matrix in a substantially-center portion of the ultrasound board 631. In addition, the plurality of pin terminals 6312 are electrically connected to the US cable C2 via the FPC connectors 6311 and the aforementioned plurality of flexible boards, and electrically connected to the ultrasound cable 31 when the ultrasound cable 31 is connected to the ultrasound connector 63.

As illustrated in FIG. 3 or 7, the frame member 632 is formed by a cylindrical metal member, and is a portion mechanically-connected to a connector on the ultrasound cable 31 side. In addition, the frame member 632 supports the ultrasound board 631 in an aperture portion on one end side (the outer casing 61 inner side).

As illustrated in FIG. 7, the electrical connection member 633 is formed by a metal member having an L-shaped cross-section, and in a posture in which a portion 6331 on one end side of the L-shaped cross-section faces the surface of the ultrasound board 631 (surface on the outer casing 61 inner side), a portion 6332 on the other end side of the L-shaped cross-section is connected to the frame member 632. In addition, an insulating sheet 6333 is provided between the portion 6331 on one end side of the L-shaped cross-section in the electrical connection member 633, and the surface of the ultrasound board 631 (the plurality of pin terminals 6312).

In addition, one end side of the US cable C2 is fixed to the portion 6331 on one end side of the L-shaped cross-section in the electrical connection member 633.

As illustrated in FIG. 7, the spacer 634 is a metal member (shield member) having a substantially-cylindrical shape partially covering an outer rim portion of the ultrasound board 631, and is fixed to one end side of the frame member 632 (the outer casing 61 inner side).

As partially illustrated in FIG. 4 or 5, the first metal member 64 is a metal frame for reinforcement that extends in the front and rear direction (Y-axis direction) in the outer casing 61, and supports the first and second circuit boards Cb1 and Cb2 and the like as mentioned above. In addition, the first metal member 64 is connected to the same electric potential as the patient GND on the endoscope observation apparatus 4 side.

FIG. 8 is a perspective view illustrating a state in which the second metal member 65 is attached to the ultrasound connector 63 illustrated in FIG. 7.

As illustrated in FIG. 8, the second metal member 65 is formed in a container shape, and is fixed by a screw or the like to the frame member 632 so as to cover part of an outer rim portion of the ultrasound board 631 (outer rim portion not covered by the spacer 634), and the surface of the ultrasound board 631. In other words, the ultrasound board 631 is shielded almost completely by its periphery being covered by the frame member 632, the electrical connection member 633, the spacer 634, and the second metal member 65, and has a structure strong to exogenous noise.

In addition, the aforementioned frame member 632, the electrical connection member 633, the spacer 634, and the second metal member 65 are connected to the same electric potential as a grounding GND on the ultrasound observation apparatus 3 side via the ultrasound cable 31.

As illustrated in FIG. 8, in a side surface portion of the second metal member 65, a cutout portion 651 for inserting one end side of the US cable C2 into the second metal member 65 is formed in a side surface portion facing the electrical connection member 633 when the second metal member 65 is fixed to the frame member 632.

FIG. 9 is a perspective view illustrating a state in which the insulating member 66 is attached to the ultrasound connector 63 illustrated in FIG. 8.

The insulating member 66 is a member formed of insulating material, and electrically-insulating the first and second metal members 64 and 65 from each other. In addition, as illustrated in FIG. 9, the insulating member 66 includes an insulating member main body 661 and a protection member 662.

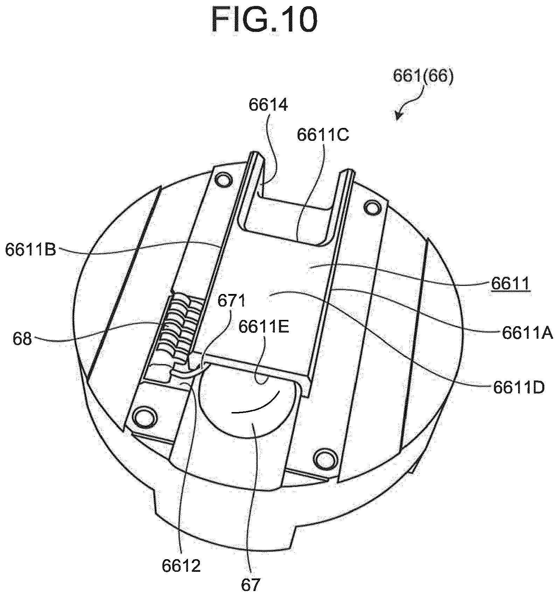

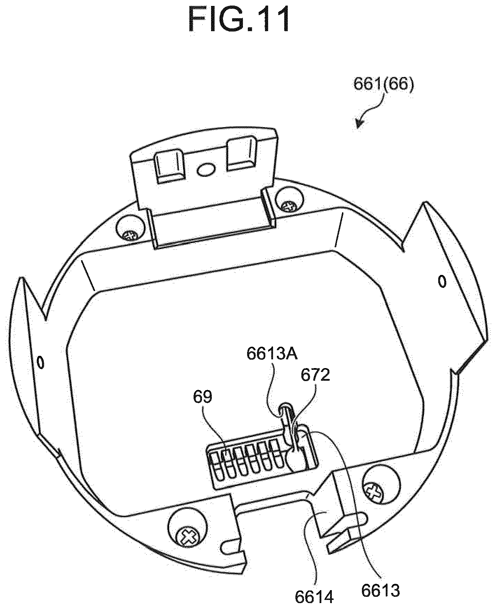

FIG. 10 is a perspective view of the insulating member main body 661 viewed from the outer casing 61 inner side. FIG. 11 is a perspective view of the insulating member main body 661 viewed from the ultrasound connector 63 side.

As illustrated in FIGS. 9 to 11, the insulating member main body 661 has a bottomed cylindrical shape, and is fixed by a screw or the like to the frame member 632 via the spacer 634 or the second metal member 65 in a state in which part of the ultrasound connector 63 is accommodated thereinside. In a state in which part of the ultrasound connector 63 is accommodated in the insulating member main body 661, the insulating member main body 661 covers the entire second metal member 65. In addition, in a state in which the ultrasound connector 63 is attached to the attachment hole 612A, the insulating member main body 661 is interposed (sandwiched) between the first and second metal members 64 and 65, to electrically insulate the first and second metal members 64 and 65 from each other.

As illustrated in FIG. 10 or 11, in the insulating member main body 661, a storage portion 6611 (FIG. 10), a first recess portion 6612 (FIG. 10), a second recess portion 6613(FIG. 11), and a cutout portion 6614 are formed in a bottom portion.

The storage portion 6611 is formed on a lateral surface (surface on the outer casing 61 inner side) of the bottom portion of the insulating member main body 661. As illustrated in FIG. 10, the storage portion 6611 includes first and second wall portions 6611A and 6611B protruding from the lateral surface and extending parallel to each other, a third wall portion 6611C protruding from the lateral surface and connecting between the first and second wall portions 6611A and 6611B, and a block portion 6611D extending parallel to the lateral surface, and installed across the first to third wall portions 6611A to 6611C. In other words, the storage portion 6611 includes an aperture portion 6611E on a side separated from the third wall portion 6611C, and is configured to be able to store a predetermined member through the aperture portion 6611E.

In addition, as illustrated in FIG. 10, a capacitor 67 is provided in the storage portion 6611 through the aperture portion 6611E.

In addition, the first and second wall portions 6611A and 6611B are formed to extend in the same direction as the portion 6331 on one end side of the L-shaped cross-section in the electrical connection member 633, in a state in which the insulating member main body 661 is fixed to the frame member 632.

As illustrated in FIG. 10, the first recess portion 6612 is a recess portion formed on the lateral surface of the bottom portion of the insulating member main body 661, and is formed at a position adjacent to the aperture portion 6611E.

In addition, as illustrated in FIG. 10, a first finger 68 functioning as a first conduction member according to the present invention is fixed to the first recess portion 6612. In addition, one leg 671 of the capacitor 67 provided in the storage portion 6611 is pulled out to the outside of the storage portion 6611 through the aperture portion 6611E, and fixed by soldering or the like to the first finger 68 fixed to the first recess portion 6612.

As illustrated in FIG. 11, the second recess portion 6613 is a recess portion formed on a medial surface (surface of the ultrasound connector 63 side) of the bottom portion of the insulating member main body 661, and is formed at a position proximate to the third wall portion 6611C. In addition, a communication hole 6613A communicated with the inside of the storage portion 6611 is formed in the second recess portion 6613.

Then, as illustrated in FIG. 11, a second finger 69 functioning as a second conduction member according to the present invention is fixed to the second recess portion 6613. In addition, the other leg 672 of the capacitor 67 provided in the storage portion 6611 is pulled out to the inside of the second recess portion 6613 through the communication hole 6613A, and fixed by soldering or the like to the second finger 69 fixed to the second recess portion 6613.

In addition, in a state in which the insulating member main body 661 is fixed to the frame member 632, the second finger 69 contacts the second metal member 65 to be electrically connected to the second metal member 65. In addition, in a state in which the ultrasound connector 63 is attached to the attachment hole 612A, the first finger 68 contacts the first metal member 64 to be electrically connected to the first metal member 64. In other words, the first and second metal members 64 and 65 are electrically connected via the capacitor 67 in a high-frequency manner.

The above-described insulating member main body 661 covers one leg 671 of the capacitor 67 and the first finger 68 when viewed from the ultrasound connector 63 side (FIG. 11). In addition, the insulating member main body 661 covers the other leg 672 of the capacitor 67 and the second finger 69 when viewed from the outer casing 61 inner side (FIG. 10). In other words, the insulating member main body 661 has a function as a coating portion according to the present invention.

In addition, almost the entire insulating member main body 661 is formed so as to have a thickness of 0.4 mm or more.

As illustrated in FIG. 10 or 11, the cutout portion 6614 is a portion in which part of the side surface portion of the insulating member main body 661 is cut out, and is formed at a position facing the cutout portion 651 of the second metal member 65 in a state in which the insulating member main body 661 is fixed to the frame member 632. In addition, one end side of the US cable C2 is inserted into the insulating member main body 661 (the second metal member 65) through the cutout portion 6614 (651), and is fixed to the portion 6331 on one end side of the L-shaped cross-section in the electrical connection member 633.

The protection member 662 is a member for protecting one end side of the US cable C2 that is inserted into the insulating member main body 661 and the second metal member 65 through the cutout portions 6614 and 651. As illustrated in FIG. 9, the protection member 662 has an L-shaped cross-section, and is fixed by a screw or the like to the frame member 632 in a state in which the US cable C2 fixed to the electrical connection member 633 is bent upward in FIG. 9 (the outer casing 61 inner side) in a portion 6621 on one end side of the L-shaped cross-section, and is further bent in a portion 6622 on the other end side of the L-shaped cross-section so as to follow the outer surface of the block portion 6611D. In other words, one end side of the US cable C2 fixed to the electrical connection member 633 is brought into a state of being fixed in a posture having a substantially-U-shape (illustrated using a broken line in FIG. 9), by the protection member 662, and the US cable C2 can be protected in a state in which a bending R regulation of a substantially-U-shaped portion, and the regulated bending R are maintained.

In the endoscope connector 6 according to the above-described present embodiment, the first and second metal members 64 and 65 are electrically insulated from each other by interposing the insulating member 66 between the first and second metal members 64 and 65. In addition, by fixing the capacitor 67 and the first and second fingers 68 and 69 to the insulating member 66, when the ultrasound connector 63 is attached to the attachment hole 612A, the first and second metal members 64 and 65 are electrically connected via the capacitor 67 in a high-frequency manner.

Thus, the endoscope connector 6 according to the present embodiment brings about such an effect that the first and second metal members 64 and 65 can be electrically connected via the capacitor 67 in a high-frequency manner only by attaching the ultrasound connector 63 to the attachment hole 612A, and assembling workability can be enhanced.

In addition, in the endoscope connector 6 according to the present embodiment, the first and second fingers 68 and 69 are employed as the first and second conduction members according to the present invention. Thus, the legs 671 and 672 of the capacitor 67 and the first and second metal members 64 and 65 can be electrically connected with certainty using a simple configuration.

In addition, in the endoscope connector 6 according to the present embodiment, the insulating member main body 661 functioning as the coating portion according to the present invention is set to have the entire thickness of 0.4 mm or more. Thus, the first and second metal members 64 and 65 can be electrically insulated from each other with certainty.

In addition, in the endoscope connector 6 according to the present embodiment, the insulating member main body 661 is formed in a container shape in which part of the ultrasound connector 63 is accommodated. Thus, only by fixing the insulating member main body 661 to the frame member 632, the other leg 672 (second finger 69) of the capacitor 67 and the second metal member 65 can be electrically connected. Thus, assembling workability of the endoscope connector 6 can be further enhanced.

### (Other Embodiments)

A mode for carrying other the present invention has been described so far. Nevertheless, the present invention is not to be limited only by the aforementioned embodiment.

In the aforementioned embodiment, the endoscope system 1 has both of a function for generating an ultrasound image, and a function for generating an endoscope image. Nevertheless, the endoscope system 1 is not limited to this configuration, and the endoscope system 1 may have a configuration only haying either one of the functions.

In the aforementioned embodiment, the insulating member main body 661 is formed in a container shape. Nevertheless, the insulating member main body 661 is not limited to this configuration, and may have a configuration only including a bottom portion by omitting a side surface portion of the insulating member main body 661, for example, as long as the first and second metal members 64 and 65 can be electrically insulated from each other. In addition, the storage portion 6611 is formed on the lateral surface of the bottom portion of the insulating member main body 661. Nevertheless, the storage portion 6611 is not limited to this configuration, and may have a configuration formed on the medial surface of the bottom portion of the insulating member main body 661.

In the aforementioned embodiment, only one capacitor 67 is provided. Nevertheless, the number of capacitors 67 is not limited to this. As another configuration, two or more capacitors 67 may be provided as long as the capacitors 67 are connected in parallel.

In the aforementioned embodiment, the endoscope system 1 is not limited to the medical field, and may be used in the industrial field, and used as an endoscope system that observes the inside of a subject such as a machine structure.

### Reference Signs List

1 ENDOSCOPE SYSTEM
2 ENDOSCOPE
3 ULTRASOUND OBSERVATION APPARATUS
4 ENDOSCOPE OBSERVATION APPARATUS
5 DISPLAY DEVICE
6 ENDOSCOPE CONNECTOR
21 INSERTION PORTION
22 OPERATING UNIT
23 UNIVERSAL CABLE
31 ULTRASOUND CABLE
41 VIDEO PROCESSOR
42 LIGHT SOURCE DEVICE
61 OUTER CASING
62 PLUG PORTION
63 ULTRASOUND CONNECTOR
64 FIRST METAL MEMBER
65 SECOND METAL MEMBER
66 INSULATING MEMBER
67 CAPACITOR
68, 69 FIRST AND SECOND FINGERS
211 ULTRASOUND PROBE
212 RIGID MEMBER
213 CURVE PORTION
214 FLEXIBLE TUBE PORTION
221 CURVE KNOB
222 OPERATING MEMBER
223 PROCESSING TOOL INSERTION PORT
611 BEND PROTECTION MEMBER
612 PROTRUDING PORTION
612A ATTACHMENT HOLE
621, 622 FIRST AND SECOND ELECTRICAL CONNECTOR PORTIONS
621A, 622A FIRST AND SECOND ELECTRICAL CONTACT POINTS
623 LIGHT GUIDE CAP
631 ULTRASOUND BOARD
632 FRAME MEMBER
633 ELECTRICAL CONNECTION MEMBER
651 CUTOUT PORTION
661 INSULATING MEMBER MAIN BODY
662 PROTECTION MEMBER
671, 672 LEGS OF CAPACITOR
6311 FPC CONNECTOR
6312 TERMINAL
6331, 6332 PORTIONS
6333 INSULATING SHEET
6611 STORAGE PORTION
6611A TO 6611C FIRST TO THIRD WALL PORTIONS
6611D BLOCK PORTION
6611E APERTURE PORTION
6612, 6613 FIRST AND SECOND RECESS PORTIONS
6614 CUTOUT PORTION
6621, 6622 PORTIONS
C1 imaging cable
C2 US CABLE
C3 SIGNAL CABLE
C11 FIRST IMAGING CABLE
C12 SECOND IMAGING CABLE
Cb1 FIRST CIRCUIT BOARD
Cb2 SECOND CIRCUIT BOARD
Fp FLEXIBLE BOARD
Fg FINGER
J1, J2 JUMPER LINES
Pi FIXING PIN
R1, R2 LUG TERMINALS
Rb RELAY BOARD
Sc SCREW
Sh1, Sh3, Sh4 SHIELD MEMBERS
Sh2 SHIELD CASING

## Claims

1. An endoscope connector comprising:
an outer casing including an attachment hole configured to communicate between an inside and an outside of the outer casing;
an electrical connector attached to the attachment hole;
a first metal member disposed in the outer casing;
a second metal member attached to the electrical connector;
an insulating member placed between the first metal member and the second metal member in a state in which the electrical connector is attached to the attachment hole, the insulating member electrically insulating the first metal member and the second metal member from each other;
a capacitor fixed to the insulating member;
a first conduction member fixed to the insulating member and electrically connected to one leg of the capacitor, the first conduction member contacting the first metal member to be electrically connected to the first metal member in the state in which the electrical connector is attached to the attachment hole; and
a second conduction member fixed to the insulating member and electrically connected to another leg of the capacitor, the second conduction member contacting the second metal member to be electrically connected to the second metal member in the state in which the electrical connector is attached to the attachment hole.

2. The endoscope connector according to claim 1, wherein the first conduction member is formed by a finger.

3. The endoscope connector according to claim 1 or 2,
wherein the second conduction member is formed by a finger.

4. The endoscope connector according to any one of claims 1 to 3, wherein the insulating member includes a coating portion configured to coat at least the one leg or the other leg of the capacitor.

5. The endoscope connector according to claim 4, wherein a thickness of the coating portion is 0.4 mm or more.

6. The endoscope connector according to any one of claims 1 to 5,
wherein the insulating member is formed in a container shape in which part of the electrical connector is accommodated, and
the second conduction member contacts the second metal member fixed to the electrical connector and is electrically connected to the second metal member in a state in which the part of the electrical connector is accommodated in the insulating member.
